# EUROPÄISCHE PATENTANMELDUNG

(11) **EP 3 181 194 A1**
(43) Veröffentlichungstag der Anmeldung: **21.06.2017**
(21) Anmeldenummer: 16200724.9
(22) Anmeldetag: 25.11.2016
(51) Int. Cl.: A61N 1/375

(54) **DURCHFÜHRUNG EINES MEDIZINELEKTRONISCHEN GERÄTS, VERFAHREN ZUR HERSTELLUNG EINER SOLCHEN UND MEDIZINELEKTRONISCHES GERÄT**

(30) Priorität: 15.12.2015 DE 102015121818
(71) Anmelder: BIOTRONIK SE & Co. KG, 12359 Berlin (DE)
(72) Erfinder: Kronmüller, Daniel, 90409 Nürnberg (DE); Eck, Stefan, 91315 Höchstadt (DE)
(74) Vertreter: Galander, Marcus

(57) **Zusammenfassung**

Durchführung eines, insbesondere implantierbaren, medizinelektronischen Gerätes, welches ein Gerätegehäuse aufweist, in dem elektronische und/oder elektrische Funktionseinheiten untergebracht sind und das eine mit der Durchführung verschlossene Gehäuseöffnung hat, wobei die Durchführung einen Isolierkörper, einen den Isolierkörper umfassenden und an der Gehäuseöffnung halternden Durchfiihrungs-Flansch und mindestens ein den Isolierkörper durchstoßendes Anschlusselement zum externen Anschluss mindestens eines Bauelements des Gerätes aufweist, wobei das oder mindestens ein Anschlusselement mindestens teilweise, insbesondere im Wesentlichen vollständig, aus einer Formgedächtnislegierung besteht.

## Beschreibung

Die Erfindung betrifft eine Durchführung eines implantierbaren medizinelektronischen Gerätes sowie auch ein derartiges Gerät. Dieses umfasst typischerweise ein Gerätegehäuse, in dem elektronische und elektrische Funktionseinheiten untergebracht sind. Eine derartige Durchführung umfasst einen Isolierkörper, insbesondere aus Keramik oder Glas, einen den Isolierkörper umfassenden Durchführungs-Flansch und mindestens ein den Isolierkörper durchstoßendendes Anschlusselement zum externen Anschluss eines elektrischen oder elektronischen Bauelements des Geräts. Zudem betrifft die Erfindung ein medizinelektronisches Gerät sowie ein Verfahren zur Herstellung eines solchen. Des Weiteren betrifft die Erfindung ein Steckerteil einer medizinelektronischen Baueinheit, welches einen Isolierkörper und mindestens ein den Isolierkörper durchstoßendes Anschlusselement zum externen Anschluss einer elektrischen Leitung der Baueinheit aufweist, sowie eine entsprechende Baueinheit und ein Verfahren zu deren Herstellung.

Implantierbare Geräte der oben bezeichneten Art sind insbesondere als Herzschrittmacher, implantierbare Kardioverter (speziell Defibrillatoren) oder auch als Cochlearimplantate seit langem in massenhaftem Einsatz. Es kann sich hierbei aber auch um eine weniger komplexe Vorrichtung, wie etwa eine Elektroden- oder Sensorleitung handeln. Medizinelektronische Baueinheiten im Sinne der nachfolgenden Ausführungen sind etwa Elektrodenleitungen zum Einsatz mit Herzschrittmachern oder implantierbaren Defibrillatoren, Nerven- und Hirnstimulatoren, Sensorleitungen o. ä.

Die meisten praktisch bedeutsamen implantierbaren medizinelektronischen Geräte sind dazu vorgesehen, über geeignet platzierte Elektroden elektrische Impulse an reizbares Körpergewebe abzugeben. Ferner können viele Geräte Signale des Nervengewebes im Körper des Patienten gezielt messen und über einen längeren Zeitraum aufzeichnen oder auswerten, um eine individuell angepasste Therapie zu wählen und den Erfolg der Behandlung in vivo zu überprüfen.

Um diese Funktionen auszuführen, sind im Gehäuse des Gerätes elektronische/elektrische Funktionseinheiten zur Erzeugung und zum Regeln der Impulse und zum Messen von Reizen untergebracht. Außen am Gerät sind Elektroden oder Anschlüsse für mindestens eine Elektrodenleitung vorgesehen, in deren distalem Endabschnitt die Elektroden zur Impulsübertragung an das Gewebe angebracht sind.

Hierfür muss eine elektrische Verbindung zwischen den im Gehäuseinneren angeordneten elektrischen und/oder elektronischen Bauteilen und der jeweiligen Elektrodenleitung hergestellt werden. Diese elektrische Verbindung wird in der Regel mittels einer Durchführung und/oder einem sogenannten Header realisiert. Eine derartige Durchführung sorgt dabei für mindestens eine elektrische Verbindung zwischen dem Inneren des Gehäuses und dem Äußeren und schließt gleichzeitig das Gehäuse des Implantats hermetisch ab. Der über der Durchführung befestigte Header führt die elektrische Verbindung der Durchführung weiter zu einer Kontaktstelle und dient zum Einstecken der mindestens einen Elektrodenleitung in eine entsprechende, meist genormte Buchse. An den Kontaktstellen der Buchse wird so ein elektrischer Kontakt zwischen dem Implantat und dem Anschlussstück der Elektrodenleitung hergestellt. Eine Durchführung und ein Header können auch in einem einzigen Bauteil realisiert sein. Auch in diesem Fall wird ein solches kombiniertes Bauteil im Folgenden allgemein als Durchführung bezeichnet.

Verbreitet sind insbesondere Durchführungen, welche mittels eines Hartlötprozesses (brazing process) aus den verschiedenen Komponenten gefügt werden.
Der Isolationskörper der Durchführung besteht im Wesentlichen aus Keramik oder Glas. Der Flansch, welcher zum hermetischen Abschluss des Gehäuses oder Implantates mit der Durchführung benötigt wird, besteht üblicherweise aus einem Metall (z.B. Titan) oder einer Legierung (z.B. Ti-6A1-4V). Als vorteilhaft wird es angesehen, Flanschmaterial und Gehäuse- bzw. Implantatmaterial aus artgleichen Werkstoffen herzustellen um diese leichter miteinander fügen zu können. Üblicherweise wird der Flansch der Durchführung mit dem Gehäuse verschweißt.

Die Kontaktelemente durchstoßen den Isolationskörper und sind voneinander und gegen den Flansch elektrisch isoliert. Sie bestehen üblicherweise aus gut leitenden Metallen (Tantal, Niob, Titan, Platin) oder Legierungen (Ptlr, FeNi, 316L). Für Die Herstellung von Kontaktelementen werden häufig Drahtabschnitte sogenannte Stifte verwendet. Die Details zur Herstellung von zusammengesetzten, weichlötbaren Kontaktelementen und deren Varianten sind z. B. in EP 2 371 418 A2 offenbart.

Derartige bekannte Durchführungen erfüllen weitgehend die an sie gestellten Anforderungen an Gasdichtheit und Biokompatibilität.

Es ist auch bekannt, bei Durchführungen oder Headern medizinelektronischer Geräte Einsätze aus Formgedächtnismaterial vorzusehen, wie etwa in der US 2002/0165588 A1 oder der US 2014/0161973 A1 gelehrt.

Die Anschluss- bzw. Kontaktelemente müssen relativ zum Durchführungs-Flansch an definierten Positionen liegen, damit sie nach der Herstellung der Durchführung in Folgeprozessen (Schweißen, Weichlöten, Crimpen) an die anderen Bauteile (Platine, Header) des med. Implantates angebunden werden können. Eine zu starke Abweichung von der Position der Kontaktelemente führt dazu, dass das Bauteil nicht verarbeitet werden kann. So kann z.B. bei mehrpoligen Durchführungen eine Addition von Toleranzen dazu führen, dass nicht alle Kontaktstellen über den Gegenkontakt liegen oder die Anschlusselemente sich nicht in einer Ebene treffen.

Um die Position im weiterverarbeitenden Prozess zu gewährleisten, können folgende Maßnahmen ergriffen werden:
- Festlegung enger Positions- bzw. Fertigungstoleranzen der Kontaktelemente während Herstellung, Transport und Weiterverarbeitung der Durchführung
- Spezialverpackungen zum Schutz der Stifte der Durchführungen
- Manuelle Nacharbeit der Stifte (Biegen, Positionieren) vor dem Weiterverarbeiten
- Sortierprüfungen vor dem Weiterverarbeiten

Das Einhalten der Positions- und Fertigungstoleranzen der Kontaktelemente relativ zum Flansch über den gesamten Produktionsprozess stellt eine große fertigungstechnische bzw. logistische Herausforderung dar. Sind die Toleranzen sehr eng, so muss die Durchführung im Produktionsablauf mit speziell hergestellten Fertigungshilfsmitteln und Transportbehältern gehandelt werden. Verpackung und Transportbehälter der Durchführungen müssen zusätzlich so konzipiert werden, dass die Position und die Toleranzen der Kontaktelemente relativ zum Flansch durch das Handling oder durch Erschütterung und Lagerbedingung nicht beeinträchtigt werden. Mit der Anzahl der Stifte sowie der Anzahl der Handlings- und Lagerprozesse steigt die Gefahr von Ausschuss. Um eine hohe Ausbeute zu erreichen, sind zusätzliche Prozess- bzw. Prüfschritte zu integrieren. Damit nur fehlerfreie Durchführungen in den nachfolgenden Montageprozess gelangen, müssen Sortierprüfungen bzw. zusätzliche Nacharbeitsschritte im Produktionsablauf integriert werden. Der zusätzliche Aufwand während der Produktion der Durchführung muss eingepreist werden und verteuert so die Durchführung bzw. das medizinische Implantat.

Der Erfindung liegt daher die Aufgabe zu Grunde, eine verbesserte Durchführung eines medizinelektronischen Gerätes bzw. ein verbessertes Steckerteil einer medizinelektronischen Baueinheit bereitzustellen, welche/welches geringeren fertigungstechnischen, Prüf- und Handling-Aufwand bei der Endmontage des Gerätes bzw. der Baueinheit erfordert und somit zu einer Senkung der Produktkosten führt. Es soll weiterhin ein geeignetes Herstellungsverfahren eines entsprechenden Gerätes bzw. einer Baueinheit angegeben werden.

Diese Aufgabe wird in ihren Vorrichtungsaspekten durch eine Durchführung mit den Merkmalen des Anspruchs 1 bzw. durch ein medizinelektronisches Gerät mit den Merkmalen des Anspruchs 9 und gemäß einem relativ unabhängigen Aspekt der Erfindung durch ein Steckerteil mit den Merkmalen des Anspruchs 12 und eine medizinelektronische Baueinheit mit den Merkmalen des Anspruchs 14 gelöst. In ihren Verfährensaspekten wird die Aufgabe durch Verfahren mit den Merkmalen des Anspruchs 10 bzw. des Anspruchs 15 gelöst. Zweckmäßige Fortbildungen sind Gegenstand der jeweiligen abhängigen Ansprüche.

Es ist ein Gedanke der Erfindung, einen Weg zur Produktion von hermetisch dichten Steckverbindern auf Basis von Metall-Keramikverbundwerkstoffen aufzuzeigen. Die Erfindung schließt weiter den Gedanken ein, den bekannten Formgedächtniseffekt im Kontext medizinelektronischer Geräte oder Baueinheiten zu nutzen, um fertigungstechnisch bedingte Positionsfehler oder durch Handhabungen vor der Endmontage hervorgerufene Positionsverschiebungen oder Formänderungen wesentlicher Teile, insbesondere der Kontaktelemente im Bezug zum Flansch, auszugleichen bzw. rückgängig zu machen. Sie schließt weiter den Gedanken ein, dieses Konzept speziell auf mindestens einen Teil der Anschlusselemente der Durchführung eines medizinelektronischen Gerätes oder des Steckerteils einer medizinelektronischen Baueinheit anzuwenden. Letztlich ist erfindungsgemäß vorgesehen, dass in der Durchführung bzw. dem Steckerteil (insbesondere in hermetisch dichter Ausführung) das oder mindestens ein Anschlusselement mindestens teilweise aus einer Formgedächtnislegierung besteht.

Hiermit wird eine Durchführung bzw. ein Steckerteil bereitgestellt, deren/dessen Anschlusselement(e) unempfindlich gegen Positionsabweichungen im Herstellungsprozess sowie gegen Verbiegen bzw. anderweitiges Verformen ist. Des Weiteren wird ein Prozess bereitgestellt, der Durchführungen bzw. Steckerteile mit verbogenen oder deformierten Anschluss- bzw. Kontaktelementen "heilt", so dass diese wieder innerhalb der vorgegebenen Toleranzbereiche liegen und ohne Qualitätseinbußen und weitere manuelle Nacharbeitungsschritte bei der Endmontage des Gerätes oder der Baueinheit eingesetzt werden können.

Mit der Erfindung lassen sich, jedenfalls in zweckmäßigen Ausgestaltungen, einer oder mehrere der nachfolgend genannten Vorteile erreichen:
- Prozessschritte beim Lieferanten und Kunden können entfallen bzw. zusammengelegt werden.
- Reduktion von Ausschuss, durch thermischen Nachbehandlungsprozess.
- Bessere Entformbarkeit der Durchführungen aus den Fertigungshilfsmitteln, durch gezieltes Entspannen der Durchführung.

In einer Ausführung der Erfindung besteht das oder mindestens ein Anschlusselement mindestens teilweise aus einer einen Einweg-Formgedächtniseffekt zeigenden Formgedächtnislegierung. In einer alternativen oder mit der erwähnten Ausführung auch kombinierbaren weiteren Ausführung besteht das oder mindestens ein Anschlusselement mindestens teilweise aus einer einen Zweiweg-Formgedächtniseffekt zeigenden Formgedächtnislegierung.

In einer weiteren Ausführung der Erfindung ist vorgesehen, dass das oder mindestens ein Anschlusselement aus mindestens zwei Teilen gefügt ist und mindestens eines der Teile vollständig aus einer Formgedächtnislegierung besteht. In einer Ausgestaltung weist das oder mindestens ein Anschlusselement ein Außenrohr und einen Kern auf und der Kern besteht aus einer Formgedächtnislegierung. Des Weiteren kann vorgesehen sein, dass das oder mindestens ein vollständig aus einer Formgedächtnislegierung bestehendes Anschlusselement oder der aus einer Formgedächtnislegierung bestehende Abschnitt des oder eines Anschlusselementes eine dünne Beschichtung aufweist.

In einer weiteren Ausführung weist die oder mindestens eine Formgedächtnislegierung zur Bildung eines Anschlusselementes oder eines Abschnitts hiervon superelastische Eigenschaften auf.

Neben den eigentlichen Anschluss- bzw. Kontaktelementen kann das erfinderische Konzept auch auf den Masseanschluss bzw. Massepin eines elektromedizinischen Gerätes oder einer Baueinheit angewandt werden. Demnach weist die Durchführung oder das Steckerteil einen Massepin auf, der mindestens abschnittsweise aus einer Formgedächtnislegierung, insbesondere einer solchen, die superelastisches Verhalten zeigt, gebildet ist.

Die vorgeschlagene Verbesserung bezieht sich letztlich auf ein medizinelektronisches Gerät, insbesondere ausgebildet als Herzschrittmacher, implantierbarer Kardioverter oder Cochlear-Implantat, oder eine medizinelektronische Baueinheit, insbesondere ausgebildet als implantierbare Elektrodenleitung.

Das erfindungsgemäße Verfahren zeichnet sich dadurch aus, dass die fertig montierte Durchführung bzw. des montierten Steckerteils vor der Montage des Gerätes bzw. der Baueinheit auf eine Temperatur oberhalb der charakteristischen Phasenumwandlungstemperatur erwärmt wird, insbesondere in einer Klimakammer oder durch Widerstandserwärmung oder über Wärmeleitung von einem angepressten Heizelement. Bezüglich des oben erwähnten Masseanschlusses kann in einer speziellen Verfahrensführung ein in thermischen Kontakt mit dem Durchführungs-Flansch (oder einer entsprechenden Stecker-Hülse oder einem Stecker-Flansch) stehender Massepin durch induktive Erwärmung des Durchführungs-Flansches erhitzt werden.

Vorteile und Zweckmäßigkeiten der Erfindung ergeben sich im Übrigen aus der Beschreibung von Ausführungsbeispielen anhand der Figuren. Von diesen zeigen:
- Fig. 1: eine schematische, teilweise geschnittene Darstellung eines implantierbaren medizinelektronischen Geräts,
- Fig. 2: eine schematische Darstellung (Schnittansicht) eines Durchführungs-Flansches herkömmlicher Bauart,
- Fig. 3A bis 3C: skizzenartige Darstellungen zur Erläuterung einer ersten Ausführungsvariante der Erfindung,
- Fig. 4A bis 4D: skizzenartige Darstellungen zur Erläuterung einer zweiten Ausführungsvariante der Erfindung,
- Fig. 5: eine schematische perspektivische Darstellung einer Ausführungsform des erfindungsgemäßen Steckerteils und
- Fig. 6: eine schematische Längsschnittdarstellung einer Ausführungsform einer erfindungsgemäßen Durchführung.

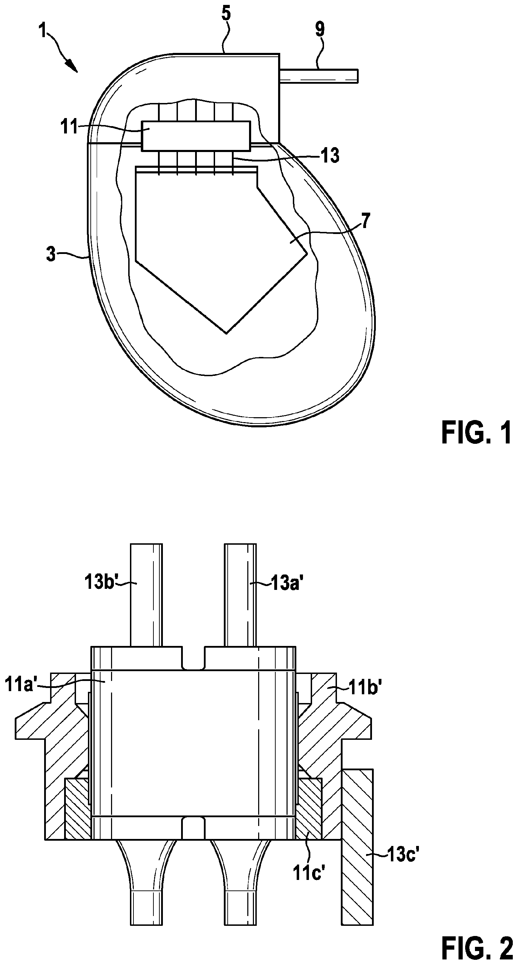

Fig. 1 zeigt einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in der Leiterplatte gesteckt und mit dieser weich verlötet. Das Verlöten kann bei einer Löttemperatur von beispielsweise 230°C geschehen.

Fig. 2 zeigt in einer Schnittdarstellung längs einer mittigen Schnittebene eine Durchführung 11' mit herkömmlichem Aufbau, die einen Keramik-Isolationskörper 11a' und einen aus Vollmaterial gedrehten Durchführungs-Flansch 11b' umfasst, der den Isolationskörper umschließt. In einer den Isolationskörper ringförmig umgebenden Aussparung an der Unterseite des Durchführungs-Flansches 11b' ist ein Lotring 11c' eingelegt; dort wird der Isolationskörper mit dem Durchführungs-Flansch mittels eines Hartlötverfahrens hermetisch dicht verbunden. Lange und kurze Anschlussstifte 13a', 13b' durchstoßen den Isolationskörper 11a', und ein Massepin 13c' ist draußen an den Durchführungs-Flansch 15' angeschweißt. Eine umlaufende Bördelkante am Durchführungs-Flansch 15' dient als Schweißkante, wenn der Flansch in eine Passung bzw. Bohrung eines (nicht dargestellten) Gerätegehäuses eingesetzt und dort verschweißt wird.

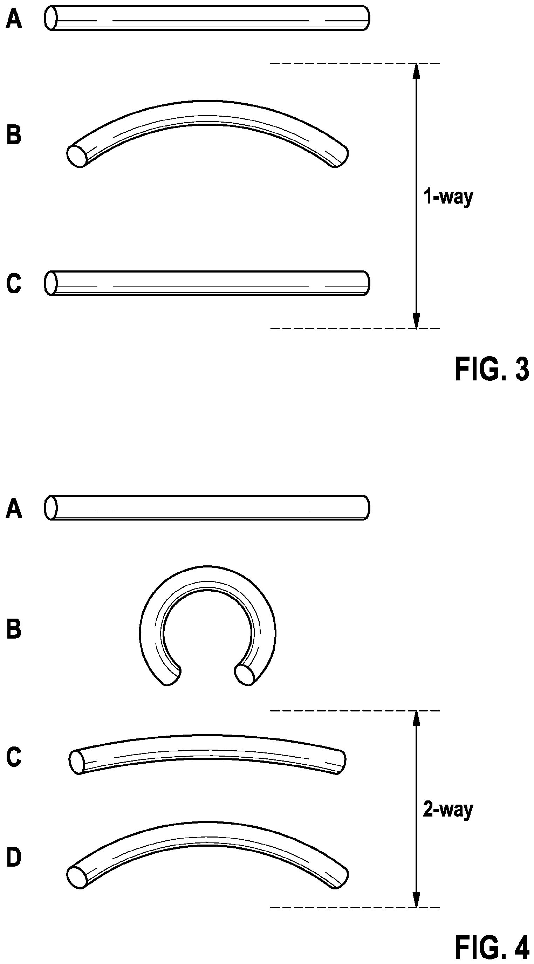

Die Figuren 3A bis 3C und 4A und 4D zeigen jeweils skizzenartig verschiedene Zustände eines als Anschlusselement dienenden zylindrischen Stiftes aus einer Formgedächtnislegierung (z.B. NiTi bzw. Nitinol, NiTiCu, CuZnAl, CuAINi, FeMnSi, FeNiCoTi), der in einer erfindungsgemäß ausgestalteten Durchführung bzw. einem Steckerteil eingesetzt sein kann. Die Darstellungen dienen zur Verdeutlichung von dessen Form bzw. Formänderungen und mechanischem Verhalten, unabhängig von der konkreten Einbausituation in eine Durchführung bzw. ein Steckerteil und ohne Beachtung von Einflüssen der Einbausituation auf die Formänderungen und das mechanische Verhalten.

In Fig. 3A ist der Stift im Anlieferungszustand und wird zu einer Durchführung prozessiert. Während des Fügens wird die ursprünglich eingestellte Temperatur des Formgedächtnis-Drahtes um einige Grad nach oben verschoben. Wie in Fig. 3B symbolisiert, kann der verlötete Stift aufgrund von Verbiegung oder Deformation beschädigt werden. Die Durchführung mit Stift ist bei einer Prüfung auf Einhaltung der Positions/Formtoleranzen als Ausschuss zu qualifizieren, da das Kontaktelement nicht spezifikationsgerecht ausgeführt ist und in den Folgeprozessen keine zuverlässige Anbindung an die Kontakte erfolgen kann.

Wie in Fig. 3C symbolisiert, kann der verformte Stift durch Nutzung des Einweg-Formgedächtniseffektes durch Erwärmen in seine ursprüngliche Form zurückgeführt und damit die innerhalb des Toleranzbereiches liegende Position seines anzuschließenden Endes wieder hergestellt werden. Die Wärmebehandlung erfolgt in einem Umluftofen oder in einer Klimakammer. Vorteilhaft ist es, die Wärmebehandlung mit einem Folgeprozess zu koppeln (Wärmebehandlung durch Vorheizen im Reflowprozess, Plasmareinigen bzw. Plasmaaktivieren vor der Weiterverarbeitung).

Auch Fig. 4A zeigt den Stift im Anlieferungszustand, wie er zu einer Durchführung bzw. einem Steckerteil prozessiert werden kann. Die veränderte Form/Endflächenposition des Stifts wird gemäß Fig. 4B in das Material trainiert (Wärmebehandlung). Anschließend wird der Stift montagegerecht verformt und in dem in Fig. 4C gezeigten Zustand in die Durchführung bzw. das Steckerteil eingefügt. Anschließend erfolgt ein Fügen, z. B. bei ca. 800°C. Während des Fügens wird die ursprünglich eingestellte Temperatur des Formgedächtnis-Drahtes um einige Grad nach oben verschoben.

In Folge des Zweiweg-Formgedächtniseffektes geht der Stift während seines Erkaltens in die definierte Form/Position über, die vorher antrainiert wurde. Die antrainierte Formänderung kann wiederholt dazu genutzt werden, Stifte aus Bestückungsvorrichtung zu entstücken. Nach dem Entformen werden die Stifte mit einem Wärmebehandlungsprozess in ihre endgültige Form überführt.

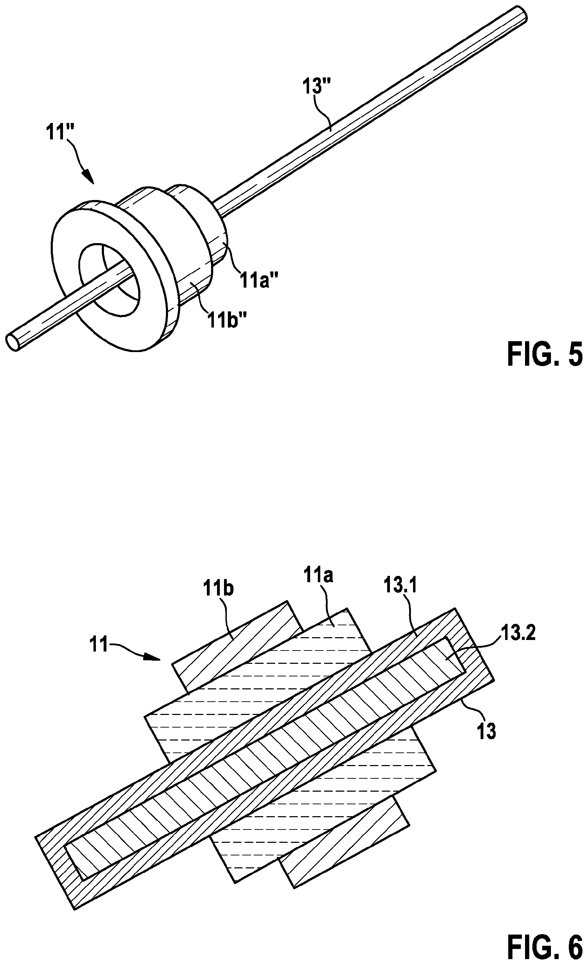

Fig. 5 zeigt in perspektivischer Ansicht ein Steckerteil 11 ", beispielsweise als Komponente einer Elektrodenleitung, das einen Isolierkörper 11a", einen den Isolierkörper umgebenden Stecker-Flansch 11b" und einen den Isolierkörper zentral durchstoßenden Anschlusstift 13" aus einer Formgedächtnislegierung umfasst.

Fig. 6 zeigt in einer schematischen Längsschnittdarstellung eine Durchführung 11, die einen Isolierkörper 11a, einen den Isolierkörper umgebenden Durchführungs-Flansch 11b und einen den Isolierkörper zentral durchstoßenden Anschlussstift 13 umfasst. Der Anschlussstift 13 ist zweiteilig aus einem Kern 13.1 aus einer Formgedächtnislegierung und einem Außenrohr 13.2 aus einem herkömmlichen Leitermetall aufgebaut.

Die Ausführung der Erfindung ist auch in einer Vielzahl von Abwandlungen der hier gezeigten Beispiele und weiter oben hervorgehobenen Aspekte der Erfindung möglich.

## Patentansprüche

1. Durchführung (11; 11') eines, insbesondere implantierbaren, medizinelektronischen Gerätes (1), welches ein Gerätegehäuse (3) aufweist, in dem elektronische und/oder elektrische Funktionseinheiten (7) untergebracht sind und das eine mit der Durchführung verschlossene Gehäuseöffnung hat, wobei die Durchführung einen Isolierkörper (11a, 11a'), einen den Isolierkörper umfassenden und an der Gehäuseöffnung halternden Durchführungs-Flansch (11b, 11b') und mindestens ein den Isolierkörper durchstoßendes Anschlusselement (13, 13a', 13b') zum externen Anschluss mindestens eines Bauelements des Gerätes aufweist,
**dadurch gekennzeichnet, dass** das oder mindestens ein Anschlusselement mindestens teilweise, insbesondere im Wesentlichen vollständig, aus einer Formgedächtnislegierung besteht.

2. Durchführung nach Anspruch 1, wobei das oder mindestens ein Anschlusselement (13, 13a', 13b') mindestens teilweise aus einer einen Einweg-Formgedächtniseffekt zeigenden Formgedächtnislegierung besteht.

3. Durchführung nach Anspruch 1 oder 2, wobei das oder mindestens ein Anschlusselement (13, 13a', 13b') mindestens teilweise aus einer einen Zweiweg-Formgedächtniseffekt zeigenden Formgedächtnislegierung besteht.

4. Durchführung nach einem der vorangehenden Ansprüche, wobei das oder mindestens ein Anschlusselement (13, 13a', 13b') aus mindestens zwei Teilen gefügt ist und mindestens eines der Teile vollständig aus einer Formgedächtnislegierung besteht.

5. Durchführung nach Anspruch 4, wobei das oder mindestens ein Anschlusselement (13) ein Außenrohr (13.2) und einen Kern (13.1) aufweist und der Kern aus einer Formgedächtnislegierung besteht.

6. Durchführung nach einem der vorangehenden Ansprüche, wobei das oder mindestens ein vollständig aus einer Formgedächtnislegierung bestehendes Anschlusselement (13, 13a', 13b') oder der aus einer Formgedächtnislegierung bestehende Abschnitt des oder eines Anschlusselementes eine dünne Beschichtung aufweist.

7. Durchführung nach einem der vorangehenden Ansprüche, wobei die oder mindestens eine Formgedächtnislegierung zur Bildung eines Anschlusselementes (13, 13a', 13b') oder eines Abschnitts hiervon superelastische Eigenschaften aufweist.

8. Durchführung nach einem der vorangehenden Ansprüche, welche einen Massepin (13c') aufweist, der mindestens abschnittsweise aus einer Formgedächtnislegierung, insbesondere einer solchen, die superelastisches Verhalten zeigt, gebildet ist.

9. Medizinelektronisches Gerät (1) mit einer Durchführung (11, 11') nach einem der vorangehenden Ansprüche, insbesondere ausgebildet als Herzschrittmacher, implantierbarer Kardioverter oder Cochlear-Implantat.

10. Verfahren zur Herstellung eines Gerätes (1) nach Anspruch 9, wobei die fertig montierte Durchführung (11; 11') vor der Montage des Gerätes auf eine Temperatur oberhalb der charakteristischen Phasenumwandlungstemperatur der Formgedächtnislegierung erwärmt wird, insbesondere in einer Klimakammer oder durch Widerstandserwärmung oder über Wärmeleitung von einem angepressten Heizelement.

11. Verfahren nach Anspruch 10, wobei ein in thermischen Kontakt mit dem Durchführungs-Flansch (11b') stehender Massepin (13c') durch induktive Erwärmung des Durchführungs-Flansches erhitzt wird.

12. Steckerteil (11") einer medizinelektronischen Baueinheit, welches einen Isolierkörper (11a") und mindestens ein den Isolierkörper durchstoßendes Anschlusselement (13 ") zum externen Anschluss einer elektrischen Leitung der Baueinheit aufweist, wobei das oder mindestens ein Anschlusselement mindestens teilweise, insbesondere im Wesentlichen vollständig, aus einer Formgedächtnislegierung besteht.

13. Steckerteil nach Anspruch 12, wobei das oder mindestens ein Anschlusselement (13") mindestens teilweise aus einer einen Einweg-Formgedächtniseffekt zeigenden oder einen Zweiweg-Formgedächtniseffekt zeigenden Formgedächtnislegierung besteht und/oder, das oder mindestens ein Anschlusselement aus mindestens zwei Teilen gefügt ist und mindestens eines der Teile vollständig aus einer Formgedächtnislegierung besteht.

14. Medizinelektronische Baueinheit mit einem Steckerteil (11 ") nach Anspruch 12 oder 13, insbesondere ausgebildet als implantierbare Elektrodenleitung.

15. Verfahren zur Herstellung einer Baueinheit nach Anspruch 14, wobei das fertige Steckerteil (11") vor der Montage der Baueinheit auf eine Temperatur oberhalb der charakteristischen Phasenumwandlungstemperatur der Formgedächtnislegierung erwärmt wird, insbesondere in einer Klimakammer oder durch Widerstandserwärmung oder über Wärmeleitung von einem angepressten Heizelement.
